# EUROPEAN PATENT APPLICATION

(11) **EP 2 851 079 A1**
(43) Date of publication of application: **25.03.2015**
(21) Application number: 12876488.3
(22) Date of filing: 13.12.2012
(51) Int. Cl.: A61K 36/537, A61P 1/16, A61P 13/12, A61P 29/00, A61P 35/00

(54) **PHARMACEUTICAL COMPOSITION CONTAINING HUMAN CYCLOOXYGENASE AND DOXORUBICIN OR DOXORUBICIN ANALOGUE, PREPARATION METHOD THEREFOR AND USE THEREOF IN PREPARING DRUGS**

(30) Priority: 10.05.2012 CN 201210142501
(71) Applicant: Lin, Xiuying, Guizhou 561000 (CN)
(72) Inventor: Lin, Xiuying, Guizhou 561000 (CN)
(74) Representative: Kramer Barske Schmidtchen Patentanwälte PartG mbB
(86) International application number: PCT/CN2012/086605
(87) International publication number: WO 2013/166833

(57) **Abstract**

Provided are a pharmaceutical composition containing human cyclooxygenase and doxorubicin or a doxorubicin analogue using *Salvia plectranthoides* (also referred to as rare grass) as a crude drug, a preparation method therefor and the use thereof in preparing drugs for treating nephritis, cholecystitis and gallbladder polyps, tumours or antiphlogistic and analgesic drugs.

## Description

### Technical field

This invention relates to a type of medical combination extracted from natural plant, in particular a type of medical combination containing natural human epoxidase (COX-2) and natural doxorubicin (atm) or natural para-doxorubicin, the method of preparation of this type of medical combination, and application of this medical combination in preparation of drugs that treat nephritis, cholecystitis, gall bladder polypus, or tumor, or anti-inflammatory drug.

### Background of the invention

Chemical name of doxorubicin is (2R,4S)-4-(3-Amino-2,3,6-trideoxy-α-L-lyxo-hexopyranosyloxy)-2-hydroxyacetyl-1,2,3, 4-tetrahydro-2,5,12-trihydroxy-7-methoxynaphthacene-6,11-dione. It is an antitumor antibiotic that can suppress synthesis of RNA and DNA, and has strongest suppression of RNA. It has relatively wide antitumor spectrum and effect on various tumors. It belongs to cell cycle nonspecific agent and can kill tumor cells of various growth cycles. It is mainly applicable to acute leucemia and is effective to both acute lymphocytic leukemia and granulocyte leucemia. For malignant lymphoma, it can be the first drug used in alternative use. It is effective to some extent in treatment of mammary cancer, fleshy tumor, lung cancer, and bladder cancer etc., and is normally used along with other antitumor drugs.

This product is a wide spectrum antitumor drug that can generate wide biochemical effect on the human body and has strong cytotoxicity. Its main acting mechanism is to be embedded in DNA so as to suppress synthesis of nucleic acid. It is clinically used to treat acute lymphocytic leukemia, acute granulocytic leukemia, Hodgkin and non-Hodgkin lymphoma, mammary cancer, lung cancer, oophoroma, soft tissue fleshy tumor, osteogenesis fleshy tumor, striated muscle fleshy tumor, embryoma of kidney, nerve mother cell tumor, bladder tumor, thyroid tumor, chorionepithelioma, prostatic cancer, testes cancer, cancer of stomach, and liver cancer etc.

Human epoxidase (cyclooxygenase COX), also prostaglandenhyperoxidesynthase (PGHS), is a rate-limiting enzyme that converts arachidonic acid to prostaglandin and eicosylene. There are two types of epoxidase with different structure and different physiological functions, i.e. COX-1 and COX-2. Human epoxidase (COX-2) was found in 1988. Natural human epoxidase was separated from plant in 1991. Natural human epoxidase is a type of protein and also a type of induced enzyme, which can function as an enzyme to accelerate generation of some chemical signal. It is active in case of inflammation and pain. When activity of COX-2 is suppressed, pain will lessen. Activated COX-2 can help generation of arachidonic acid, generating various prostaglandins, participating in various physiological processes and pathologic processes of the human body. At present, a well recognized opinion is that COX-2 may participate in tumor generation and development through promotion of cell proliferation, suppression of cell withering, promotion of angiopoiesis, and suppression of immunologic function.

Although there have been many papers of research on human epoxidase (COX-2), prior art has not found a natural plant that contains rich amount of COX-2, which can adopt the method of traditional Chinese medicine to realize industrial production of natural doxorubicin (atm) and human epoxidase (COX-2).

### References

Xue Peini, Chang Xiaohong, Yang Lirong, Journal of Yanan University (medical science) 2011 Volume 09 Session 03: "Progress in research of correlation between COX-2 and non-small cell carcinoma of the lung";
Zou Yingying, Yao Nan, Wang Fang, Gao Qian, Song Jinglin, Journal of Kunming Medical College 2010 Volume 31 Session 01: "Characteristics of expression of CDX2 and COX2 in human large intestine cancer and their relation with metastasis";
Zhang Huifeng, Gao Xiaoqin, Li Rongshan, Journal of Shanxi Medical University 2009 Volume 40 Session 12: "P_(38)MAPK/COX_2 Experimental research in protection effect of signal transduction path in second window of pre-treatment of rat kidney ischemia";
Shi Bainian, Liu Jiang, He Jiangfang, Dai Licheng, Zhou Jianfang, Zhao Weifeng, Gan Jianhe, Zhongyuan Medical Journal 2007 Volume 34 Session: "Expression of epoxidation enzyme 2 in liver injury of hepatic failure model"

### Summary of the invention

To compensate aforesaid shortcomings of prior art, the purpose of this invention is to provided a type of medical combination containing natural human epoxidase (COX-2) and natural doxorubicin (atm) or natural para-doxorubicin, the method of preparation of this type of medical combination, and application of this medical combination in preparation of drugs that treat nephritis, cholecystitis, gall bladder polypus, or tumor, or anti-inflammatory drug. This invention provides a medical combination prepared from natural medical plant rich in human epoxidase (COX-2) and natural doxorubicin or para-doxorubicin, and this medical combination can realize medical industrialization of natural doxorubicin (atm) and human epoxidase (COX-2) using traditional Chinese medicine method. Many side effects of chemical synthesized drug can be avoided and good treatment effect is provided.

The technical scheme of this invention is: a type of medical combination containing natural human epoxidase (COX-2) and natural doxorubicin (atm) or natural para-doxorubicin, wherein raw material of this combination is treasures grass (also referred to as Guizhou treasures grass).

In this technical scheme, "raw material of this combination is treasures grass" means that powder obtained from dewatering, drying, and crushing of whole treasures grass (including roots) or fresh treasures grass or extract of treasures grass can be used.

Said treasures grass extract refers to one of the following:
Fresh treasures grass vibrated to wool;
Water decoction liquid of treasures grass;
Powder obtained from concentration and drying of treasures grass water decoction liquid; Alcohol extraction liquid of treasures grass water decoction liquid obtained by alcohol extract;
Powder obtained from concentration and drying of alcohol extraction liquid of treasures grass water decoction liquid obtained by alcohol extract;
Extract obtained from organic extraction of whole treasures grass or treasures grass water decoction liquid;
Powder obtained from re-concentration and drying of extract obtained from organic extraction of whole treasures grass or treasures grass water decoction liquid;
Extract obtained from CO₂ supercritical extraction of whole treasures grass or treasures grass water decoction liquid;
Power obtained from concentration and drying of the extract obtained from CO₂ supercritical extraction of whole treasures grass or treasures grass water decoction liquid; and
Volatile oil extracted from whole treasures grass.

In addition to aforesaid raw material treasures grass or its extract, this medical combination containing natural human epoxidase (COX-2) and natural doxorubicin (atm) or natural para-doxorubicin may also contain auxiliary materials permitted in the field of pharmacy. For example, excipient, condiment, or antioxidant used to make tablets; antioxidant or stabilizer used to make capsules; condiment, stabilizer, or antioxidant used to make oral liquid or syrup; emulsifier or vaseline used to make externally applied medicinal extract; and emulsifier, solvent, or stabilizer used to make injection.

Plant classification position of said treasures grass (provisional name) in above technical scheme is as follows:
Guizhou treasures grass:
Saivia plectranthoides Griff
Angiospermae
Dicotyledoneae
Sympetalae
Tubiflorae
Verbenineae
Labiatae
Salvia linn

This treasures grass is an annual or perennial herb, with fibrous root system, and erect or rising repent rhizome. The repent stem has rootage (single or cluster) at its node. The stem has the shape of obtuse quadrangle (square), has grooves, and is covered by thick short lanugo.

The seeds of this treasures grass (provisional name) had been delivered to China Typical Cultures Preservation Center at Wuhan University for preservation on April 2, 2011. Preservation number is CCTCC No: P201102 (refer to Attachment 1 of the Summary of Invention). New species right of this plant has been applied to USA: Take-up No.: 12/215.016; USA Trademark Take-up No.: 776M688.

According to National Center for Drug Screening: sample No.nc00168954 containing doxorubicin (or para-doxorubicin, i.e. with doxorubicin resistance) is effective (refer to Attachment 2 of the Summary of Invention). According to National Center for Drug Screening: Human epoxidase-2 (COX-2) activity suppression report; Assessment of experiment results of effect of antitumor substance on immunity biological activity, proliferation of T and B lymphocyte, and cytotoxicity (refer to Attachment 2 of the Summary of Invention). According to screening carried out by Hangzhou High Throughput Drug Screening Center, this plant contains 7 types of cancer cell suppression active substance (refer to Attachment 3 of the Summary of Invention).

Information of this plant species has been submitted to Ministry of Agriculture and completed.

The technical scheme of the second task of this application is the method to prepare said medical combination containing natural human epoxidase (COX-2) and natural doxorubicin (atm) or natural para-doxorubicin, wherein the steps are as follows:
(1) Cleaning of whole treasures grass;
(2) Dewatering, drying, and crushing of the clean whole treasures grass obtained in Step (1) to yield a powder;

Said step (2) can also adopt treasures grass extract obtained by one of the following:
(2) -1 Treasures grass water decoction liquid or powder obtained by re-concentration and drying of this liquid;
(2) -2 Alcohol extraction liquid of treasures grass water decoction liquid obtained by alcohol extract, or powder obtained by re-concentration and drying of the same;
(2) -3 Extract of whole treasures grass or treasures grass water decoction liquid obtained by organic extraction, or powder obtained by re-concentration and drying of this extract;
(2) -4 Extract obtained by CO₂ supercritical extraction of whole treasures grass or treasures grass water decoction liquid, or powder obtained by re-concentration and drying of this extract;
(2) -5 Volatile oil extracted from whole treasures grass
(3) Mix powder and/or volatile oil obtained in step (2), (2) -1, (2) -2, (2) -3, (2) -4, or (2) -5 with medical auxiliary substance to prepare required dose pattern;
(4) Sterilization;
(5) Analysis;
(6) Packing

The scheme of the third task of this application is: application of said medical combination containing natural human epoxidase (COX-2) and natural doxorubicin (atm) or natural para-doxorubicin in preparation of drugs that treat nephritis;

The scheme of the fourth task of this application is: application of said medical combination containing natural human epoxidase (COX-2) and natural doxorubicin (atm) or natural para-doxorubicin in preparation of drugs that treat cholecystitis and gall bladder polypus;

The scheme of the fifth task of this application is: application of said medical combination containing natural human epoxidase (COX-2) and natural doxorubicin (atm) or natural para-doxorubicin in preparation of drugs that treat tumor;

The scheme of the sixth task of this application is: application of said medical combination containing natural human epoxidase (COX-2) and natural doxorubicin (atm) or natural para-doxorubicin in preparation of anti-inflammatory drug.

When said medical combination containing natural human epoxidase (COX-2) and natural doxorubicin (atm) or natural para-doxorubicin is used to prepare drugs to treat nephritis, cholecystitis, or tumor, or anti-inflammatory drug, the administration mode may be: oral administration tablet, capsule, oral liquid or syrup, oral administration distillate, externally applied eye drops, externally applied eye ointment, externally applied scald pain alleviating ointment, and externally applied anti-inflammation, hemostasis, stimulation disperse, and pain alleviating powder. Injection is not excluded.

Administration dose: for adults, equivalent to 5 g - 6 g of Guizhou treasures grass powder per day; this application recommends 5.4 g per day, that is, three times a day and 1.8 g each time.

Treatment period (course of treatment) is 7 days. Two courses are required to treat ordinary phlogosis. Six courses are required to treat chronic phlogosis. Six or more courses are normally required to treat nephritis, cholecystitis, or tumor.

The medical combination of this invention can quickly treat phlogosis, eliminate swelling, and relieve pain. It can also remove virus, suppress tumor cells, and activate vitality of human body immune system. Therefore, the medical combination of this invention can treat chronic nephritis, acute cholecystitis (including bile regurgitation cholecystitis), and various tumors in lymph, lung, liver, stomach, and mammary gland, as well as fibroid tumor and leucemia. It can also be used to treat trachoma and pink eye.

As an externally applied drug, the medical combination of this invention can be used to treat traumatic injury, scald due to hot water or fire, and sore of diabrosis and running with pus.

Externally applied pounded paste of fresh raw material plant of the medical combination of this invention can help bone union.

The medical combination of this invention also features beautification effect and can be used for development of beautification products.

The following provides evidences of treatment effect of drugs prepared according to this invention for nephritis, cholecystitis and gall bladder polypus, and tumors:

When the medical combination containing natural human epoxidase (COX-2) and natural doxorubicin (atm) or natural para-doxorubicin is applied to prepare drugs to treat nephritis, confirmed diagnosis of the patient adopts normal standards, and "effective", "apparently effective" and "cured" etc. also adopt traditional standards. Administration dose of drug of this invention is: for adults, three times a day and 1.8 g each time; course of treatment is 7 days. After 6 or more courses, treatment effect comparison is shown in the table below:

**Table 1**

| | Effective | Apparently effective | Cured | No effect |
|---|---|---|---|---|
| Group using drug of this invention | 98.3% | 92.5% | 84.2% | |
| Group using bunitrolol | 93.2% | 59% | | |

### Curing time of the group using drug of this invention is 7 - 15 days.

When the medical combination containing natural human epoxidase (COX-2) and natural doxorubicin (atm) or natural para-doxorubicin is applied to prepare drugs to treat cholecystitis, confirmed diagnosis of the patient adopts normal standards, and "effective", "apparently effective" and "cured" etc. also adopt traditional standards. Administration dose of drug of this invention is: for adults, three times a day and 1.8 g each time; course of treatment is 7 days. After 6 or more courses, treatment effect comparison is shown in the table below:

**Table 2**

| | Effective | Apparently effective | Cured | No effect |
|---|---|---|---|---|
| Group using drug of this invention | 95.8% | 93.2% | 80% | |
| Group using Danning tablets | 82.79% | 78.57% | | 7.14% |

When the medical combination containing natural human epoxidase (COX-2) and natural doxorubicin (atm) or natural para-doxorubicin is applied to prepare drugs to treat tumor, confirmed diagnosis of the tumor (lymph tumor) patient adopts normal standards, and "effective", "apparently effective" and "cured" etc. also adopt traditional standards. Administration dose of drug of this invention is: for adults, three times a day and 1.8 g each time; course of treatment is 7 days. After 8 or more courses, treatment effect comparison is shown in the table below:

**Table 3**

| | Effective | Apparently effective | Cured | No effect |
|---|---|---|---|---|
| Group using drug of this invention | 89.3% | 80.5% | 80% | |
| Group using taxol | 48.8% | 32.6% | 16.3% | 2.3% |

When the medical combination containing natural human epoxidase (COX-2) and natural doxorubicin (atin) or natural para-doxorubicin is applied to prepare ordinary anti-inflammation pain alleviating drug, confirmed diagnosis of the phlogosis patient adopts normal standards, and "effective", "apparently effective" and "cured" etc. also adopt traditional standards. Administration dose of drug of this invention is: for adults, three times a day and 1.8 g each time; course of treatment is 7 days. After 2 or more courses, treatment effect comparison is shown in the table below:

**Table 4**

| | Effective | Apparently effective | Cured | No effect |
|---|---|---|---|---|
| Group using drug of this invention | 97.6% | 92.9% | 90% | |
| Group using streptomycin | 14.29% | 78.57% | | 7.14% |

Regarding technical effect of drugs of application of this invention, refer to Attachment 2, 3, and 4 to the Summary of Invention. Experiment report of Hangzhou High Throughput Drug Screening Center (Attachment 3 to the Summary of Invention): According to screening of treasures grass drugs, relatively strong suppression is found for cell lung cancer, fibrosarcoma, cervical carcinoma, and liver cancer; relatively weak suppression is found for carcinoma of large intestine, prostatic cancer, and mammary cancer.
(1) The Chinese medicine extraction liquid has apparent effect of suppression of cell proliferation for A549 cells and this suppression effect has dependence on drug concentration.
(2) The Chinese medicine extraction liquid has apparent effect of suppression of cell proliferation for HK9 cells and this suppression effect has dependence on drug concentration.
(3) The Chinese medicine extraction liquid has apparent effect of suppression of cell proliferation for PC3 cells and this suppression effect has dependence on drug concentration.
(4) The Chinese medicine extraction liquid has apparent effect of suppression of cell proliferation for HT1080 cells and this suppression effect has dependence on drug concentration.
(5) The Chinese medicine extraction liquid has apparent effect of suppression of cell proliferation for Hela cells and this suppression effect has dependence on drug concentration.
(6) The Chinese medicine extraction liquid has apparent effect of suppression of cell proliferation for MDA-MB-231 cells and this suppression effect has dependence on drug concentration.
(7) The Chinese medicine extraction liquid has apparent effect of suppression of cell proliferation for Hepc2 cells and this suppression effect has dependence on drug concentration.

### Experiment results:

8 grams of dry Chinese medicine extraction liquid are used to prepare 120 grams of juice at 1:15 concentration (55.56 ug/ml). This juice has apparent toxic action for cells. Under such toxic action, since cells in upper chamber die or have lowered activity, number of cells migrated to lower chamber decreases. When drug concentration is lower than 55.56 ug/ml, there is basically no toxic action for cells. (Experiment information is attached later.)

This invention provides a medical combination prepared from natural plant rich in human epoxidase (COX-2) and natural doxorubicin or para-doxorubicin. This combination can adopt traditional Chinese medicine method to realize medical industrialized manufacture of natural doxorubicin (atm) and human epoxidase (COX-2). This can avoid many side effects of chemical synthesized drugs and provide good treatment effect.

### Description of drawing figures

**Fig. 1** shows curves of dynamic detection of suppression of A549 cells out-of-strain proliferation by Chinese medicine extraction liquid and observation of suppression effect;
**Fig. 2** shows curves of dynamic detection of suppression of HT29 cells out-of-strain proliferation by Chinese medicine extraction liquid and observation of suppression effect;
**Fig. 3** shows curves of dynamic detection of suppression of PC3 cells out-of-strain proliferation by Chinese medicine extraction liquid and observation of suppression effect;
**Fig. 4** shows curves of dynamic detection of suppression of HT1080 cells out-of-strain proliferation by Chinese medicine extraction liquid and observation of suppression effect;
**Fig. 5** shows curves of dynamic detection of suppression of Hela cells out-of-strain proliferation by Chinese medicine extraction liquid and observation of suppression effect;
**Fig. 6** shows curves of dynamic detection of suppression of MDA-MB-231 cells out-of-strain proliferation by Chinese medicine extraction liquid and observation of suppression effect;
**Fig. 7** shows curves of dynamic detection of suppression of HepG2 cells out-of-strain proliferation by Chinese medicine extraction liquid and observation of suppression effect;
**Fig. 8** is a specificity atlas of compound;
**Fig. 9** shows curves of detected suppression of A549 cells out-of-strain migration capability by Chinese medicine extraction liquid;
**Fig. 10** shows curves of detected suppression of PC3 cells out-of-strain migration capability by Chinese medicine extraction liquid;
**Fig. 11** shows curves of detected suppression of HT1080 cells out-of-strain migration capability by Chinese medicine extraction liquid;
**Fig. 12** shows curves of detected suppression of Hela cells out-of-strain migration capability by Chinese medicine extraction liquid;
**Fig. 13** shows curves of detected suppression of MDA-MB-231 cells out-of-strain migration capability by Chinese medicine extraction liquid;
**Fig. 14** shows curves of detected suppression of HepG2 cells out-of-strain migration capability by Chinese medicine extraction liquid;
**Fig. 15** shows curves of detected HT29 cells out-of-strain migration capability;
**Fig. 16** shows a real-time cell analyzer;
**Fig. 17** and **Fig. 18** show measured curves that reflect cardiac muscle cells beating characteristics and status;
**Fig. 19** is a page of analysis of parameters and data in the cardiac muscle cell model;
**Fig. 20** shows a curve of effect of Chinese medicine extraction liquid on activity of first generation cardiac muscle cells of new born rat;
**Fig. 21** shows change of beating curve of first generation cardiac muscle cells with time after adding the drug;
**Fig. 22** shows curves of effect of Chinese medicine extraction liquid on beating rate;
**Fig. 23** shows curves of effect of Chinese medicine extraction liquid on amplitude;
**Fig. 24** shows IC50 curves of effect of Chinese medicine extraction liquid on bearing rate and amplitude of first generation cardiac muscle cells of new born rat.

### Preferred embodiment

Preferred embodiment 1: method to prepare tablets of mixed medical combination containing natural human epoxidase (COX-2) and natural doxorubicin (atm) or natural para-doxorubicin, and their clinic use

Method to prepare tablets of natural doxorubicin (atm) (or natural para-doxorubicin) and human epoxidase:

### Method to prepare steam distillate:

Clean and sterilize treasures grass and mix 500 g of such grass with 5 kg of water. Use steam distillation to obtain volatile oil and distillate. Reserve the volatile oil for later use. Subject the distillate to low temperature for longer than 24h, filter it to obtain supernatant, recover ethanol till there is no smell of alcohol, and concentrate to thick paste. Dry and crush the same to obtain extractum powder. Take the volatile oil and sediment, and evenly spray them on the extractum powder. Add auxiliary medical materials i.e. unitary acid calcium phosphate, lactose, starch, and magnesium stearate etc., in the form of micro-powder, to prepare corresponding dose pattern.

Method to prepare extractum powder tablets: crush dry extractum to fine powder, filter through 5-6 mesh sieve, use ethanol for wetting and pelletizing, and then carry out tabletting.

Spraying rotary pelletizing method: add unitary acid calcium phosphate, lactose, and starch and place dry extractum fine powder in a coating pan. While rotating the pan, spray mist for wetting and gradual pelletizing.

Tabletting from powder: clean, sterilize, drily prepare, and dry the drug material, and process micron powder of Chinese medicine, of grain size 10 um (1-20 um). Filter through a 300 mesh sieve to prepare tablets.

Capsule preparation method: mix 500 g of drug material with 5 kg of water 3. Method to prepare capsules: processing the drug material: clean, process, purify, sterilize, crush to 75 um 4.1, filter through 200 um mesh sieve, and fill capsules. Solvent extraction process: suitably crush treasures grass and mix 500 g of such crushed grass with 5 kg of water. Use impregnating method, percolation method and coction method. Lipotropy can be selected: petroleum ether.benzene.>diethyl ether>ethyl acetate>acetone>ethanol> methanol>water (hydrophilicity) etc., as organic solvent during extraction process.

This treasures grass galenical had been sent to Shanghai National Center for Drug Screening in 2006 for screening. Results show that this plant has natural human opoxidase-2 (cox-2) activity and immunity biological activity, and very good effect in anti-inflammation treatment. Also, it has very good effect of suppression of T and B lymphocytes, hence very good future in application.
Attachment-1 (preservation evidence, 1 page)
Attachment-2 (state new drug screening evidence, 3 pages)
Attachment-3 (Hangzhou screening evidence, 23 pages)

### Attachment-1

### China Typical Cultures Preservation Center

### Notice of acceptance (receipt) for preservation of culture used for patent procedure

Address: Wuhan University, Wuhan, China; Post code: 430072 Tel: (027) 68752319 Fax: (027) 68754733 E-mail: ccrcc@whu.ecu.cn

### Consigner and his/her agent:

Consigner: Lin Xiuying Preservation Number of this Patent agent:
Patent application No.:
Name of culture for which preservation is requested and indicated discrimination characteristics
Seeds of Guizhou treasures grass

This culture is attached with:
□ Scientific description
□ Proposed classification name

Note: Check any above box to indicate availability of the item; otherwise attach X. This culture has been received and registered by this Preservation Center on Feb.22, 2011.

According to your request, this culture will be preserved for 30 years since this date. A further five years of preservation will be allowed upon request after expiration of the 30 years.

Survivance of this culture had been checked by this Preservation Center on March 26, 2011 and the result was positive.

### China Typical Cultures Preservation Center

Signature of responsible person:
April 2, 2011

### Attachement-2

### The National Center for Drug Screening

### In-vitro screening test of anti-tumor biological activity

Screening method: Methy-Thiazol-Tetrozolium (MTT) reduction method
Cell strain: HL-60 human leucemia
Duration of action: 72h
Assessment result: No activity: 1 mg/ml < 50%;
With activity: 1 mg/ml > 50%

### Suppression rate for growth of tumor cells, %

| Concentration Sample No. | 0.5 | 0.25 | 0.13 | 0.063 | 0.031 | Assessment | Remark |
|---|---|---|---|---|---|---|---|
| NC00168954 | 41.8 | 55.9 | 72.2 | 72.9 | 38.9 | | |
| Concentration (M) | 10⁻⁴ | 10⁻⁵ | 10⁻⁶ | 10⁻⁷ | 10⁻⁸ | | |
| Doxorubicin | 70.0 | 81.6 | 79.0 | 57.6 | 0 | Effective | |

Assuming original liquid concentration of 10

Tested by: Xu Chenghui, Date: 2011.12

### The national Center for Crug Screening

### Report of suppression of activity of human epoxidase-2 (COX-2)

### Purpose of experiment:

Cyclo-oxygenase generates prostaglandin and oxygen free radical (ROS) through metabolism of arachidonic acid (AA). Cyclo-oxygenase (COX2) is a key factor of occurrence and development of phlogosis. Cyclo-oxygenase 1 (COX1) mainly controls physiological functions. While being anti-inflammatory, COX2 selective inhibitor avoids interference to physiological functions, making it a very good target point of anti-inflammatory treatment. The inhibitor used to screen it has apparent future application.

### Testing principle:

COX2 metabolizes AA to generate prostaglandin and ROS. Enzyme activity can be determined by measurement of ROS fluorescence or PGE2 generated.

### Experiment results:

COX-2 enzyme suppression rate by the sample (%)

| | | | |
|---|---|---|---|
| microg/ml | | 10 | |
| NC00127983 | | 7.42 | |
| | | | |
| microM | 0.1 | 1 | 10 |
| Celecoxib | 34.05 | 70.55 | 92.10 |

Tested by: Jin Daozhong

Date: June 11, 2006

### The national Center for Crug Screening

### Immunity biological activity screening test

Name of model: Test of proliferation reaction of T and B lymphocytes and cytotoxicity Method of screening: As stimulation by mitotic source ConA,LPS, BALB/C mouse spleen lymphocyte will be subject to a series of change in its form and metabolism, so that it is converted to mother cell that undergoes differentiation and proliferation. Add 10mg/ml of LPS (lipopolysaccharide) to induce proliferation of B lymphocyte, and quantitatively measure cell proliferation using 3H-TdR infiltration method. Chondriosome deoxidizing enzyme in living cells can reduce yellow MTT to blue A peptide and output of A peptide is directly proportional to living cells. After being dissolved by organic solvent, value of OD can be measured using enzyme meter.

### Testing system: in-vitro

Result assessment: Lymphocyte proliferation adopts CPM value of measured sample deducted by CPM value of comparison well, divided by CPM value of comparison well, and timed by %. Negative sign before the percentage indicates suppression of T and B cells by this sample. Absence of negative sign before the percentage indicates proliferation of T and B cells by this sample. Lymphocytotoxicity is judged "positive" or "negative".

Without cytotoxicity, proliferation/suppression percentage of T and B lymphocytes is above 15%, indicating presence of effect.

| | Measuring concentration | | | | Cytotoxicity | | T lymphocyte | | T cell proliferation | B lymphocyte | | B cell proliferation |
|---|---|---|---|---|---|---|---|---|---|---|---|---|
| Name of sample | | | | | | | ConA stimulation | | Measuring of overall activity | LPS stimulation | | Measuring of overall activity |
| | | Mean OD | SD | P value | Yes/No | | Mean CPM | SD | Proliferation/ suppression percentage | Mean CPM | SD | Proliferation/ suppression percentage |
| | | | | | | Negative comparison | 1569 | 173 | | 1587 | 52 | |
| Comparison | | 0.287 | 0.001 | | | Positive comparison | 34014 | 2399 | | 7434 | 506 | |
| NC0012798 3 | 1:4000 | 0.243 | 0.030 | 0.1203 | No | | 30884 | 13747 | -9% | 7381 | 887 | -1% |
| | 1:400 | 0.278 | 0.032 | 0.6593 | No | | 16357 | 15445 | -52% | 5753 | 220 | -23% |
| | 1:40 | 0.410 | 0.032 | 0.0166 | No | | 7754 | 5290 | 77% | 3272 | 917 | -56% |

### Attachment-3

Hangzhou High Throughput Drug Screening Center ACEA biosciences

### Consigned service item:

### Technical research based on xCELLigence non-marking dynamic real-time cell signal and function analysis system (RTCA)

**1. Detection of cytotoxicity effect interposed by Chinese medicine extraction liquid**
**2. Detection of effect of Chinese medicine extraction liquid on cell migration Based on the technology of xCELLigence non-marking dynamic real-time cardiac muscle cell analysis system (RTCA Cardio System)**
**3. Experiment report on detection of effect of Chinese medicine extract on cardiac muscle cell**

Name of client: Lin Xiuying

Reported by: Hangzhou High Throughput Drug Screening Center

Report released on: Feb.14, 2012

### 1. Detection of cytotoxicity effect interposed by Chinese medicine extraction liquid

### (I) Project briefs

This experiment adopts xCELLigence real-time cell analysis system to detect suppression of cell proliferation by Chinese medicine extraction liquid and 1 C50 of suppression of cell proliferation by drugs. Cells adopted include A549, HT1080, Hela, MDA-MB-231, HT29, HepG2, and PC3.

### (II) Experiment instrument:

xCELLigence real-time cell analysis system jointly developed by ACEA and Roche

### (III) Experiment reagents and consumables:

DMEM culture medium: goods No.: SH30022.01B, Hyclone (China)
Improved RPMI-1640 culture medium: goods No.: SH30809.01B, Hyclone (China)
MEM culture medium: goods No.: SH30024.01B, Hyclone (China)
F12 culture medium: goods No.: SH30526.01, Hyclone (China)
MyCOA's 5A culture medium: goods No.: GNM 16600, Gino (China)
Serum: goods No.: 16000-044, Gibco (USA)
Pancreatic enzyme: goods No.: 27250-018, Gibco (USA)
PBS: goods No.: SH30256.01B, Hyclone (China)
Absorption pipette: goods No.: 4487, Costar
Centrifuge tube: goods No.: 430791, Corning
96x E-plate: ACEA
A549 (non-small cell carcinoma of the lung cell strain), PC3 (human prostatic cancer cell strain), HT1080 (human formed fibrosarcoma cell strain), Hela (human cervical carcinoma cell strain), MDA MB-231 (human mammary cancer cell strain), and HepG2 (human liver cancer cell strain): ATCC
Chinese medicine extraction liquid: provided by Lin Qiuying

### (IV) Method of drug configuration

According to information provided by client, 125 ml of medical juice of Chinese medicine extraction liquid is obtained from 20 g of the drug, i.e. a concentration of 160 mg/ml. During the experiment, this is diluted by culture medium according to actual demand, and then added into the cell.

### (V) Test design and scheme

### Testing method:

On 96x E-plate, inoculate cells according to Layout: A549 (5000 cells/well), Hela (2500 cells/well), HT1080 (2500 cells/well), MDA-MB-231 (4000 cells/well), HT29 (10000 cells/well), HepG2 (10000 cells/well), and PC3 (10000 cells/well). After a night of cells proliferation, add drug according to Layout and continue detection for 72h.

Experiment Layout is as follows:

### Plate 1

| | 1 2 | 3 4 | 5 6 | 7 8 | 9 10 | 11 12 |
|---|---|---|---|---|---|---|
| | HT29 | PC3 | MDA-MB-231 | HepG2 | Hela | HT1080 |
| | Chinese medicine extraction liquid | | | | | |
| A | 32 mg/ml | 64 mg/ml | 32 mg/ml | 32 mg/ml | 32 mg/ml | 32 mg/ml |
| B | 16 mg/ml | 21.33 mg/ml | 16 mg/ml | 16 mg/ml | 16 mg/ml | 16 mg/ml |
| C | 8 mg/ml | 7.11 mg/ml | 8 mg/ml | 8 mg/ml | 8 mg/ml | 8 mg/ml |
| D | 4 mg/ml | 2.37 mg/ml | 4 mg/ml | 4 mg/ml | 4 mg/ml | 4 mg/ml |
| E | 2 mg/ml | 0.79 mg/ml | 2 mg/ml | 2 mg/ml | 2 mg/ml | 2 mg/ml |
| F | 1 mg/ml | 0.26 mg/ml | 1 mg/ml | 1 mg/ml | 1 mg/ml | 1 mg/ml |
| G | 0.5 mg/ml | 87.79 mg/ml | 0.5 mg/ml | 0.5 mg/ml | 0.5 mg/ml | 0.5 mg/ml |
| H | con | con | con | con | con | con |

### Plate 2

| | 12 | 3 |
|---|---|---|
| | A549 | |
| | Chinese medicine extraction liquid | |
| A | 32 mg/ml | |
| B | 16 mg/ml | con |
| C | 8 mg/ml | |
| D | 4 mg/ml | |
| E | 2 mg/ml | |
| F | 1 mg/ml | |
| G | 0.5 mg/ml | |
| H | 0.25 mg/ml | |

### Acquisition signal setup is as follows:

| Step | Number of cycles | Interval/time | Remark |
|---|---|---|---|
| Step_1 | 1 | 1 min | Measurement cardinal number |
| Step_2 | 100 | 15 min | Proliferation detection curve |
| Step_3-1 | 200 | 2 min | Test of drug action |
| Step_3-2 | 300 | 15 min | Test of drug action |

### (VI) Experiment results

### 1. RTCA Plot

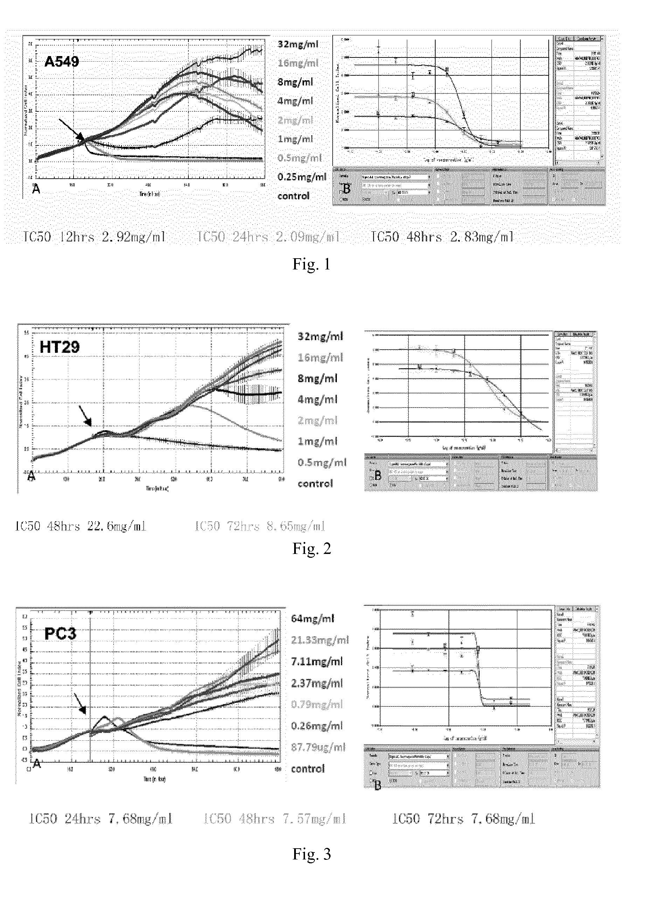

### (1) (Fig.1) Dynamic detection of suppression of A549 cells out-of-strain proliferation by Chinese medicine extraction liquid and suppression effect

Fig. 1 shows dynamic detection of suppression of A549 cells out-of-strain proliferation by Chinese medicine extraction liquid and observation of suppression effect. A is a real-time dynamic diagram of cell growth and drug effect. In this diagram, abscissa indicates time of cell proliferation and drug effect, and ordinate indicates normalized cell index (NCI). The cell index prompts cell growth status, and the higher is this value, the larger is the number of living cells. The arrow points to dosing time and the dosing concentration is shown in this diagram. This diagram shows drug concentration dependence dynamic response curve of the cell under the action of Chinese medicine extraction liquid. controll is negative comparison that shows normal cell growth dynamic curve. B shows IC50 values at drug action time points of 12h, 24h, and 48h after addition of the Chinese medicine extraction liquid inA549 cells, and the IC50 fitting curve, automatically detected and calculated by the real-time cell analysis system. It can be seen from this figure that the Chinese medicine extraction liquid apparently suppresses proliferation of A549 cells, and the suppression features drug concentration dependence.

### (2) (Fig. 2) Dynamic detection of suppression of HT29 cells out-of-strain proliferation by Chinese medicine extraction liquid and suppression effect

Fig. 2 shows dynamic detection of suppression of HT29 cells out-of-strain proliferation by Chinese medicine extraction liquid and observation of suppression effect. A is a real-time dynamic diagram of cell growth and drug effect. B shows IC50 values at drug action time points of 48h and 72h after addition of the Chinese medicine extraction liquid in HT29 cells, and the IC50 fitting curve, automatically detected and calculated by the real-time cell analysis system. It can be seen from this figure that the Chinese medicine extraction liquid apparently suppresses proliferation of HT29 cells, and the suppression features drug concentration dependence.

### (3) (Fig.3) Dynamic detection of suppression of PC3 cells out-of-strain proliferation by Chinese medicine extraction liquid and suppression effect

Fig. 3 shows dynamic detection of suppression of PC3 cells out-of-strain proliferation by Chinese medicine extraction liquid and observation of suppression effect. A is a real-time dynamic diagram of cell growth and drug effect. B shows IC50 values at drug action time points of 24h, 48h, and 72h after addition of the Chinese medicine extraction liquid in PC3 cells, and the IC50 fitting curve, automatically detected and calculated by the real-time cell analysis system. It can be seen from this figure that the Chinese medicine extraction liquid apparently suppresses proliferation of PC3 cells, and the suppression features drug concentration dependence.

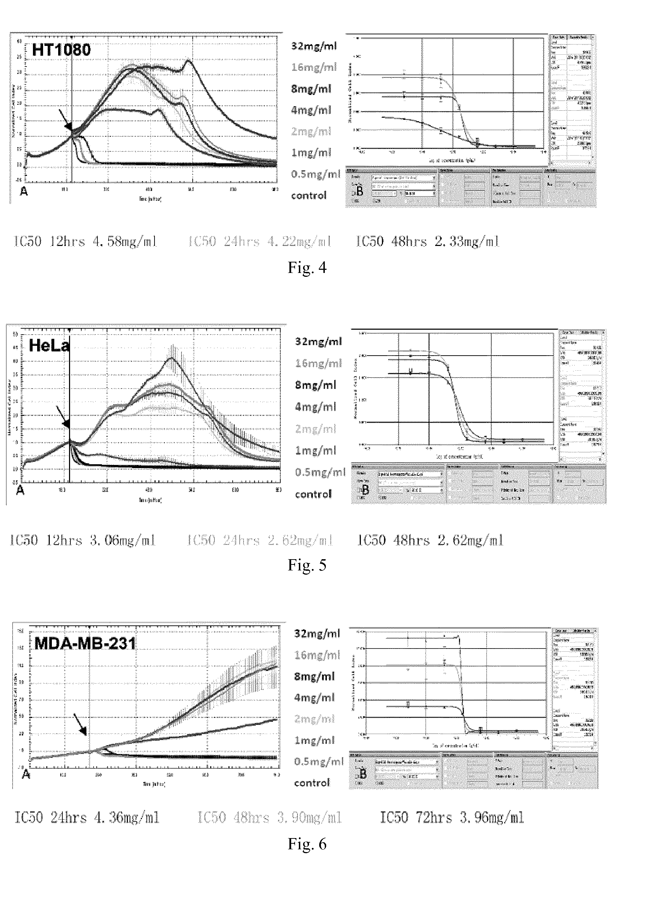

### (4) (Fig. 4) Dynamic detection of suppression of HT1080 cells out-of-strain proliferation by Chinese medicine extraction liquid and suppression effect

Fig. 4 shows dynamic detection of suppression of HT1080 cells out-of-strain proliferation by Chinese medicine extraction liquid and observation of suppression effect. A is a real-time dynamic diagram of cell growth and drug effect. B shows IC50 values at drug action time points of 12h, 24h and 48h after addition of the Chinese medicine extraction liquid in HT1080 cells, and the IC50 fitting curve, automatically detected and calculated by the real-time cell analysis system. It can be seen from this figure that the Chinese medicine extraction liquid apparently suppresses proliferation of HT1080 cells, and the suppression features drug concentration dependence.

### (5) (Fig. 5) Dynamic detection of suppression of Hela cells out-of-strain proliferation by Chinese medicine extraction liquid and suppression effect

Fig. 5 shows dynamic detection of suppression of Hela cells out-of-strain proliferation by Chinese medicine extraction liquid and observation of suppression effect. A is a real-time dynamic diagram of cell growth and drug effect. B shows IC50 values at drug action time points of 12h, 24h and 48h after addition of the Chinese medicine extraction liquid in Hela cells, and the IC50 fitting curve, automatically detected and calculated by the real-time cell analysis system. It can be seen from this figure that the Chinese medicine extraction liquid apparently suppresses proliferation of Hela cells, and the suppression features drug concentration dependence.

### (6) (Fig. 6) Dynamic detection of suppression of MDA-MB-231 cells out-of-strain proliferation by Chinese medicine extraction liquid and suppression effect

Fig. 6 shows dynamic detection of suppression of MDA-MB-231 cells out-of-strain proliferation by Chinese medicine extraction liquid and observation of suppression effect.

A is a real-time dynamic diagram of cell growth and drug effect. B shows IC50 values at drug action time points of 24h, 48h and 72h after addition of the Chinese medicine extraction liquid in MDA-MB-231 cells, and the IC50 fitting curve, automatically detected and calculated by the real-time cell analysis system. It can be seen from this figure that the Chinese medicine extraction liquid apparently suppresses proliferation of MDA-MB-231 cells, and the suppression features drug concentration dependence.

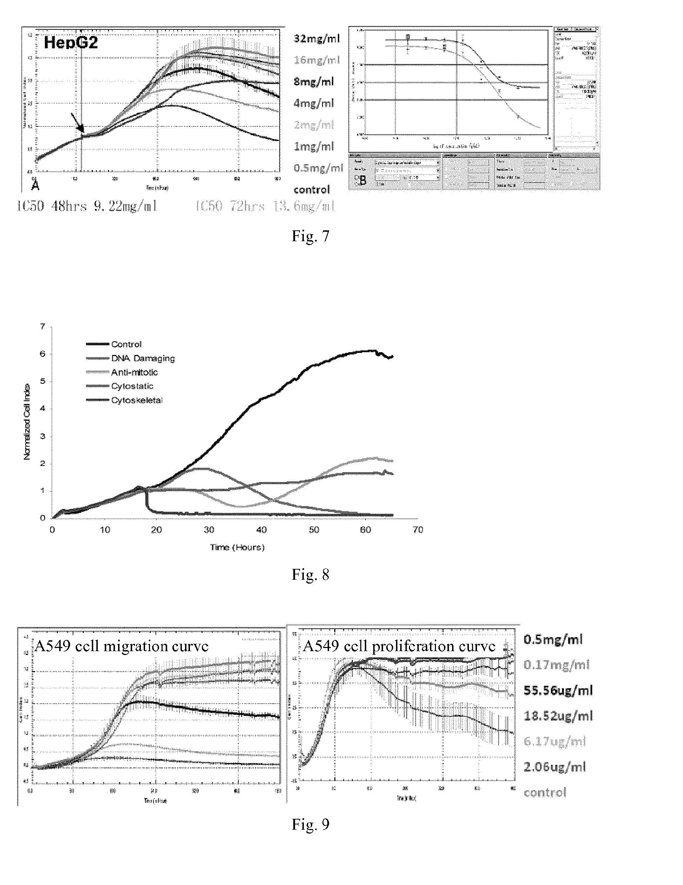

### (7) (Fig. 7) Dynamic detection of suppression of HepG2 cells out-of-strain proliferation by Chinese medicine extraction liquid and suppression effect

Fig. 7 shows dynamic detection of suppression of HepG2 cells out-of-strain proliferation by Chinese medicine extraction liquid and observation of suppression effect. A is a real-time dynamic diagram of cell growth and drug effect. B shows IC50 values at drug action time points of 48h and 72h after addition of the Chinese medicine extraction liquid in HepG2 cells, and the IC50 fitting curve, automatically detected and calculated by the real-time cell analysis system. It can be seen from this figure that the Chinese medicine extraction liquid apparently suppresses proliferation of HepG2 cells, and the suppression features drug concentration dependence.

### 2. Cytotoxicity effect of the Chinese medicine extraction liquid on cell strain

| Cell | IC50 (mg/ml) | | | |
|---|---|---|---|---|
| | 12 hrs | 24 hrs | 48 hrs | 72 hrs |
| A549 (5 K/well) | 2.92 | 2.09 | 2.83 | / |
| HT29 (10 K/well) | / | / | 22.6 | 8.65 |
| PC3 (10 K/well) | / | 7.68 | 7.57 | 7.68 |
| HT1080 (2.5 K/well) | 4.58 | 4.22 | 2.33 | / |
| Hela (2.5 K/well) | 3.06 | 2.62 | 2.62 | / |
| MDA-MB-231 (4 K/well) | / | 4.36 | 3.90 | 3.96 |
| HepG2 (10 K/well) | / | / | 9.22 | 13.6 |

### (VII) Conclusions of the experiment, possible questions, and recommendations

1. This experiment examined cytotoxicity effect of the Chinese medicine extraction liquid on cell strains of 7 different organs: human non-small cell carcinoma of lung cell strain (A549), human carcinoma of large intestine cell strain (HT29), human prostatic carcinoma cell strain (PC3), human formed fibrosarcoma cell strain (HT1080), human cervical carcinoma cell strain (Hela), human mammary cancer cell strain (MDA-MB-231), and human liver cancer cell strain (HepG2).
2. Due to different actions of the drug on different cell strains, 4 times points (12 hr, 24 hr, 48 hr, and 72 hr after dosing) are selected and IC50 values of corresponding time points have been obtained according to particular conditions of each cell.
3. The Chinese medicine extraction liquid has suppression effect on proliferation of all 7 cell strains selected, and very good concentration gradient is seen. The effect on HT1080, Hela, MDA-MB-231, and A549 cells is relatively strong, and the effect on HepG2, HT29, and PC3 cells is relatively weak.
4. From the point of view of drug development, study of the effect of this drug on lung cancer, cervical carcinoma, and mammary cancer etc. can continue, while considering an experiment to determine cytotoxicity effect of this drug on non-tumor cell strains.
5. Study of compound mechanism: According to compound specificity response atlas, the Chinese medicine has apparent cytotoxicity effect on these cell strains, and the atlas on HT29 and PC3 cells shows DNA Damage cytotoxicity effect characteristic (refer to Fig. 8).

### Fig. 8 Compound specificity atlas

Real-time impedance data from xCELLigence system provide compound specificity atlas. Such atlas depends on compound biological action mechanism. In this example, addition of compound of different biological action mechanism and target point specificity can yield special compound atlas. For compounds without specificity, such atlas can be used to set some assumptions that can be verified using forecast mode.

### Experiment records

Saved in ACEA Biosciences (Hangzhou) Co., Ltd. client database No.:1112051511P3, 1112081603P3, 1112141440P5, 1112201057P5

### 2. Test of effect of Chinese medicine extraction liquid on cell migration

### (I) Project briefs

This experiment adopts xCELLigence real-time cell analysis system to test suppression of cell migration by certain Chinese medicine extraction liquid provided by Guizhou Chinese Medicine Co., Ltd. Cells used include A549, PC3, HT1080, Hela, MDA-MB-231, and HepG2.

### (II) Experiment instruments

This experiment adopts xCELLigence DP real-time cell analysis system jointly developed by ACEA and Roche.

### (III) Experiment reagents and consumables

DMEM culture medium: goods No.: SH30022.01B, Hyclone (China)
Improved RPMI-1640 culture medium: goods No.: SH30809.01B, Hyclone (China)
MEM culture medium: goods No.: SH30024.01B, Hyclone (China)
F12 culture medium: goods No.: SH30526.01, Hyclone (China)
Pancreatic enzyme: goods No.: 27250-018, Gibco (USA)
DMSO: goods No.: D2650, Sigma (USA)
PBS: goods No.: SH30256.01B, Hyclone (China)
Absorption pipette: goods No.: 4487, Costar
Centrifuge tube: goods No.: 430791, Corning
96x E-plate: ACEA
CIM plate: ACEA
A549 (non-small cell carcinoma of the lung cell strain), PC3 (human prostatic cancer cell strain), HT1080 (human formed fibrosarcoma cell strain), Hela (human cervical carcinoma cell strain), MDA MB-231 (human mammary cancer cell strain), and HepG2 (human liver cancer cell strain): ATCC
Chinese medicine extraction liquid: provided by Lin Qiuying

### (IV) Method of drug configuration

According to information provided by client, 125 ml of medical juice of Chinese medicine extraction liquid is obtained from 20 g of the drug, i.e. a concentration of 160mg/ml. During the experiment, this is diluted by culture medium according to actual required concentration.

### (V) Experiment design and scheme

### Experiment method:

According to Layout, in the CIM plate lower chamber, add drug prepared using culture medium containing 10% of serum, and in the upper chamber, add drug prepared using serum-free medium. On DP system, measure base line. Then, in the upper chamber, add cells subject to re-suspension using serum-free medium and joint incubation with each compound for 1h. On DP, detect for 24h - 48h.

At the same time, provide parallel comparison of cell proliferation on 96x E-plate. According to the Layout, add drug prepared using culture medium containing 10% of serum, and measure base line on SP. Then, add cells subject to re-suspension using medium containing 10% of serum and joint incubation with each compound for 1h. On SP, detect for 24h - 48h.

Used cell concentrations: A549 (60 k/well), PC3 (40 k/well), HT1080 (30 k/well), Hela (30 k/well), MDA-MB-231 (80 k/well), HepG2 (80 k/well)

### Experiment Layout is given below:

| | | **Cell migration** | | | **Comparison of cell proliferation** | |
|---|---|---|---|---|---|---|
| | | Line1 | Line2 | | Line1 | Line2 |
| | | A549 60 k/well | | | A549 60 k/well | |
| A | Chinese medicine extraction liquid | 0.5 mg/ml | | | 0.5 mg/ml | |
| B | | 0.17 mg/ml | | | 0.17 mg/ml | |
| C | | 55.56 ug/ml | | | 55.56 ug/ml | |
| D | | 18.52 ug/ml | | | 18.52 ug/ml | |
| E | | 6.17 ug/ml | | | 6.17 ug/ml | |
| F | | 2.06 ug/ml | | | 2.06 ug/ml | |
| G | Control | CON | | | CON | |
| H | Serum free | SF CON | | | SF CON | |

| **DP2** | | **Cell migration** | | | **Comparison of cell proliferation** | |
|---|---|---|---|---|---|---|
| | | Line1 | Line2 | | Line1 | Line2 |
| | | PC3 40 k/well | | | PC3 40 k/well | |
| A | Chinese medicine extraction liquid | 64 mg/ml | | | 64 mg/ml | |
| B | | 21.33 mg/ml | | | 21.33 mg/ml | |
| C | | 7.11 mg/ml | | | 7.11 mg/ml | |
| D | | 2.37 mg/ml | | | 2.37 mg/ml | |
| E | | 0.79 mg/ml | | | 0.79 mg/ml | |
| F | | 0.26 mg/ml | | | 0.26 mg/ml | |
| G | Control | CON | | | CON | |
| H | Serum free | SF CON | | | SF CON | |

| **DP3** | | **Cell migration** | | | **Comparison of cell proliferation** | |
|---|---|---|---|---|---|---|
| | | Line1 Line2 | | | Line1 Line2 | |
| | | HT1080 30 k/well | | | HT1080 30 k/well | |
| A | Chinese medicine extraction liquid | 0.5 mg/ml | | | 0.5 mg/ml | |
| B | | 0.17 mg/ml | | | 0.17 mg/ml | |
| C | | 55.56 ug/ml | | | 55.56 ug/ml | |
| D | | 18.52 ug/ml | | | 18.52 ug/ml | |
| E | | 6.17 ug/ml | | | 6.17 ug/ml | |
| F | | 2.06 ug/ml | | | 2.06 ug/ml | |
| G | Control | CON | | | CON | |
| H | Serum free | SF CON | | | SF CON | |

| **DP4** | | **Cell migration** | | | **Comparison of cell proliferation** | |
|---|---|---|---|---|---|---|
| | | Line1 Line2 | | | Line1 Line2 | |
| | | Hela 30 k/well | | | Hela 30 k/well | |
| A | Chinese medicine extraction liquid | 0.5 mg/ml | | | 0.5 mg/ml | |
| B | | 0.17mg/ml | | | 0.17 mg/ml | |
| C | | 55.56 ug/ml | | | 55.56 ug/ml | |
| D | | 18.52 ug/ml | | | 18.52ug/ml | |
| E | | 6.17 ug/ml | | | 6.17 ug/ml | |
| F | | 2.06 ug/ml | | | 2.06 ug/ml | |
| G | Control | CON | | | CON | |
| H | Serum free | SF CON | | | SF CON | |

| **DP5** | | **Cell migration** | | | **Comparison of cell proliferation** | |
|---|---|---|---|---|---|---|
| | | Line1 Line2 | | | Line1 Line2 | |
| | | MDA-MB-231 80 k/well | | | MDA-MB-231 80 k/well | |
| A | Chinese medicine extraction liquid | 4 mg/ml | | | 4 mg/ml | |
| B | | 1.33 mg/ml | | | 1.33 mg/ml | |
| C | | 0.44 mg/ml | | | 0.44 mg/ml | |
| D | | 0.15 mg/ml | | | 0.15 mg/ml | |
| E | | 49.38 ug/ml | | | 49.38 ug/ml | |
| F | | 16.46 ug/ml | | | 16.46 ug/ml | |
| G | Control | CON | | | CON | |
| H | Serum free | SF CON | | | SF CON | |
| | | | | | | |

| **DP6** | | **Cell migration** | | | **Comparison of cell proliferation** | |
|---|---|---|---|---|---|---|
| | | Line1 Line2 | | | Line1 Line2 | |
| | | HepG2 80 k/well | | | HepG2 80 k/well | |
| A | Chinese medicine extraction liquid | 2 mg/ml | | | 2 mg/ml | |
| B | | 1 mg/ml | | | 1 mg/ml | |
| C | | 0.5 mg/ml | | | 0.5 mg/ml | |
| D | | 0.25 mg/ml | | | 0.25 mg/ml | |
| E | | 0.125 mg/ml | | | 0.125 mg/ml | |
| F | | 62.5 ug/ml | | | 62.5 ug/ml | |
| G | Control | CON | | | CON | |
| H | Serum free | SF CON | | | SF CON | |

### Acquisition signal setup is as follows:

| Step | Number of cycles | Interval/time | Remark |
|---|---|---|---|
| Step_1 | 1 | 1 min | Measurement cardinal number |
| Step_2 | 200 | 15 min | Cell migration curve under tested drug action |

### Experiment results

### 1. RTCA Plot

### (1) (Fig. 9) Detected suppression of A549 cells out-of-strain migration capability by Chinese medicine extraction liquid

### Fig. 9 shows detected suppression of A549 cells out-of-strain migration capability by Chinese medicine extraction liquid.

The left part of this figure shows curves of cell migration suppressed by drug. The right part of this figure is a real-time dynamic diagram of parallel test of effect of the drug on cell proliferation. The abscissa indicates time of cell culture and drug action. The ordinate indicates normalized cell index (NCI). The NCI prompts cell growth status. In the cell migration test, the higher is the NCI, the more cells migrated into the lower chamber. In the cell proliferation test, the higher is the NCI, the more living cells. Dosing concentration is shown in this figure.

The left part of this figure shows response curves of cell migration capability subject to drug concentration dependence under the action by the Chinese medicine extraction liquid. Control is negative comparison that displays normal cell migration dynamic curve.

The right part of this figure shows suppression of cell proliferation subject to drug concentration dependence under the action by the Chinese medicine extraction liquid. Control is negative comparison that displays normal cell proliferation dynamic curve.

When concentration of the drug added exceeds 55.56 ug/ml, apparent toxic action on cells exists. Under such toxic action, since cells in upper chamber die or have reduced activity, number of cells migrated to the lower chamber decreases. When drug concentration is lower than 55.56 ug/ml, basically no toxic action on cells exists, but cell migration is suppressed to some extent, subject to drug concentration dependence. Therefore, this drug has certain suppression effect on migration of A549 cells.

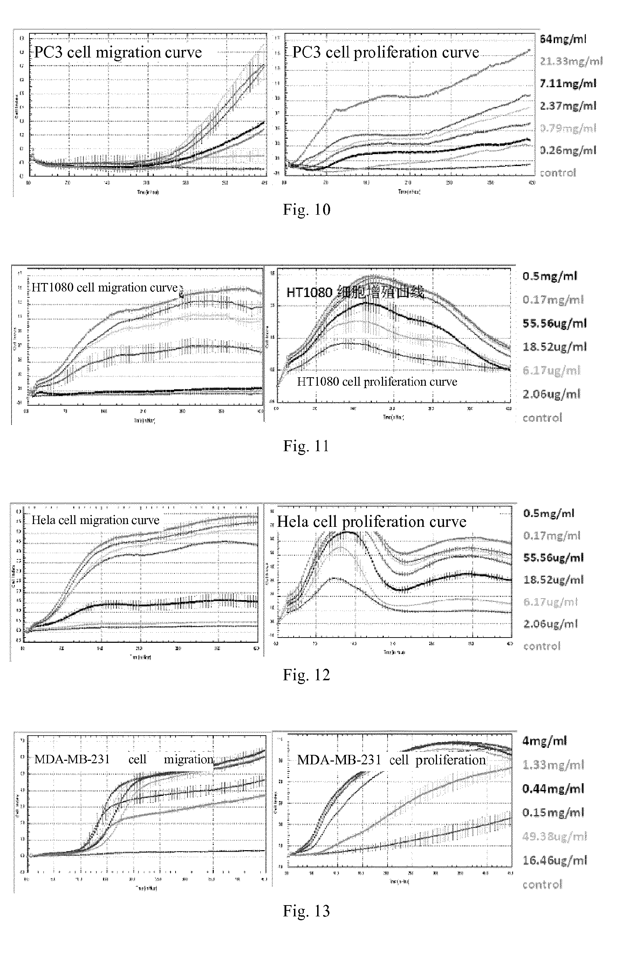

### (2) (Fig. 10) Detected suppression of PC3 cells out-of-strain migration capability by Chinese medicine extraction liquid

### Fig. 10 shows detected suppression of PC3 cells out-of-strain migration capability by Chinese medicine extraction liquid.

The left part of this figure shows curves of cell migration suppressed by drug. The right part of this figure is a real-time dynamic diagram of parallel test of effect of the drug on cell proliferation. Dosing concentration is shown in this figure.

The left part of this figure shows response curves of cell migration capability subject to drug concentration dependence under the action by the Chinese medicine extraction liquid. Control is negative comparison that displays normal cell migration dynamic curve.

The right part of this figure shows suppression of cell proliferation subject to drug concentration dependence under the action by the Chinese medicine extraction liquid. Control is negative comparison that displays normal cell proliferation dynamic curve.

This test indicates that addition of Chinese medicine extraction liquid of different concentrations has apparent toxic action on cells. Under such toxic action, since cells in upper chamber die or have reduced activity, number of cells migrated to the lower chamber decreases. However, when concentration of Chinese medicine extraction liquid is lower than 7.11 mg/ml, under action by the drug, cell migration is higher than that of control, indicating stimulation to migration.

### (3) (Fig. 11) Detected suppression of HT1080 cells out-of-strain migration capability by Chinese medicine extraction liquid

### Fig. 11 shows detected suppression of HT1080 cells out-of-strain migration capability by Chinese medicine extraction liquid.

The left part of this figure shows curves of cell migration suppressed by drug. The right part of this figure is a real-time dynamic diagram of parallel test of effect of the drug on cell proliferation. Dosing concentration is shown in this figure.

The left part of this figure shows response curves of cell migration capability subject to drug concentration dependence under the action by the Chinese medicine extraction liquid. Control is negative comparison that displays normal cell migration dynamic curve.

The right part of this figure shows suppression of cell proliferation subject to drug concentration dependence under the action by the Chinese medicine extraction liquid. Control is negative comparison that displays normal cell proliferation dynamic curve.

When concentration of the drug added exceeds 18.52 ug/ml, apparent toxic action on cells exists. Under such toxic action, since cells in upper chamber die or have reduced activity, number of cells migrated to the lower chamber decreases. When drug concentration is lower than 18.52 ug/ml, basically no toxic action on cells exists, but cell migration is suppressed to some extent, subject to drug concentration dependence. Therefore, this drug has certain suppression effect on migration of A549 cells.

### (4) (Fig. 12) Detected suppression of Hela cells out-of-strain migration capability by Chinese medicine extraction liquid

### Fig. 12 shows detected suppression of Hela cells out-of-strain migration capability by Chinese medicine extraction liquid.

The left part of this figure shows curves of cell migration suppressed by drug. The right part of this figure is a real-time dynamic diagram of parallel test of effect of the drug on cell proliferation. Dosing concentration is shown in this figure.

The left part of this figure shows response curves of cell migration capability subject to drug concentration dependence under the action by the Chinese medicine extraction liquid. Control is negative comparison that displays normal cell migration dynamic curve.

The right part of this figure shows suppression of cell proliferation subject to drug concentration dependence under the action by the Chinese medicine extraction liquid. Control is negative comparison that displays normal cell proliferation dynamic curve.

The drug added has apparent toxic action on cells. Under such toxic action, since cells in the upper chamber die or have reduced activity, number of cells migrated to the lower chamber decreases. This drug has no apparent suppression of cell migration.

### (5) (Fig. 13) Detected suppression of MDA-MB-231 cells out-of-strain migration capability by Chinese medicine extraction liquid

### Fig. 13 shows detected suppression of MDA-MB-231 cells out-of-strain migration capability by Chinese medicine extraction liquid.

The left part of this figure shows curves of cell migration suppressed by drug. The right part of this figure is a real-time dynamic diagram of parallel test of effect of the drug on cell proliferation. Dosing concentration is shown in this figure.

The left part of this figure shows response curves of cell migration capability subject to drug concentration dependence under the action by the Chinese medicine extraction liquid. Control is negative comparison that displays normal cell migration dynamic curve.

The right part of this figure shows suppression of cell proliferation subject to drug concentration dependence under the action by the Chinese medicine extraction liquid. Control is negative comparison that displays normal cell proliferation dynamic curve.

In early stage (before 20hr) of the test, added drug has certain stimulation on migration of MDA-MB-231 cells. When drug concentration is lower than 0.15 mg/ml, no apparent toxic action on cells exists. At this time, the more drug added, the stronger the stimulation to cells migration. When concentration of added drug exceeds 0.4 mg/ml, relatively apparent toxic action on cells exists. Under such toxic action, since cells in the upper chamber die or have reduced activity, number of cells migrated to the lower chamber decreases.

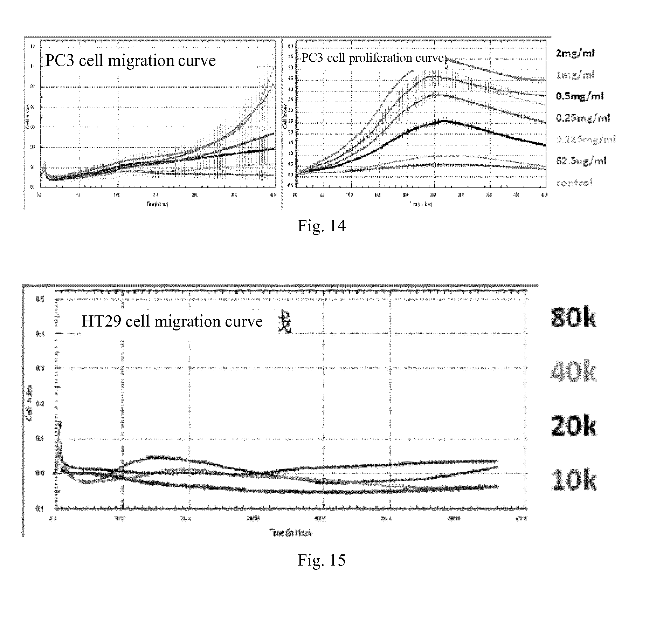

### (6) (Fig. 14) Detected suppression of HepG2 cells out-of-strain migration capability by Chinese medicine extraction liquid

### Fig. 14 shows detected suppression of HepG2 cells out-of-strain migration capability by Chinese medicine extraction liquid.

The left part of this figure shows curves of cell migration suppressed by drug. The right part of this figure is a real-time dynamic diagram of parallel test of effect of the drug on cell proliferation. Dosing concentration is shown in this figure.

The right part of this figure shows suppression of cell proliferation subject to drug concentration dependence under the action by the Chinese medicine extraction liquid. Control is negative comparison that displays normal cell proliferation dynamic curve. The drug added has apparent toxic action on cells. Under such toxic action, since cells in the upper chamber die or have reduced activity, number of cells migrated to the lower chamber decreases. This drug has no apparent suppression of cell migration.

### (7) (Fig. 15) Detected HT29 cells out-of-strain migration capability Fig. 15 shows detection of in vitro migration capability of HT29 cell strain.

As shown in this figure, when cell concentration is 80 k/well, 40 k/well, 20 k/well, and 10 k/well respectively, HT29 has no apparent cell migration capability. Therefore, suppression of cell migration capability by drug cannot be detected.

### (VII) Conclusions of the experiment, possible questions, and recommendations

1. This experiment examined suppression effect of the Chinese medicine extraction liquid on in vitro migration capability of cell strains of 7 different organs: human non-small cell carcinoma of lung cell strain (A549), human carcinoma of large intestine cell strain (HT29), human prostatic carcinoma cell strain (PC3), human formed fibrosarcoma cell strain (HT1080), human cervical carcinoma cell strain (Hela), human mammary cancer cell strain (MDA-MB-231), and human liver cancer cell strain (HepG2). At the same time, parallel test of effect of the drug on cell proliferation is carried out, to determine that suppression of cell migration capability by the drug is not due to its cytotoxicity.
2. This Chinese medicine extraction liquid of high concentration has apparent toxic action on A549 and HT1080 cells. Under such toxic action, since cells in the upper chamber die or have reduced activity, number of cells migrated to the lower chamber decreases. At low concentration, this drug has certain suppression effect on cell migration.
3. This Chinese medicine extraction liquid has apparent toxic action on Hela and HepG2 cells. Under such toxic action, since cells in the upper chamber die or have reduced activity, number of cells migrated to the lower chamber decreases. At low concentration, this drug has no apparent suppression effect on migration of these cells. This drug has no suppression of migration of these cells.
4. This Chinese medicine extraction liquid of high concentration has apparent toxic action on PC3 and MDA-MB-231 cells. Under such toxic action, since cells in the upper chamber die or have reduced activity, number of cells migrated to the lower chamber decreases. At low concentration, this drug has certain stimulation effect on migration of these cells.

### Experiment records

Saved in ACEA Biosciences (Hangzhou) Co., Ltd. client database No.: 1112211551D12, 1112211605P4, 1112271558D12, 1112271559P4, 1112311454P6, 1112311452D12, 1201101537D12, 1201101540P2, 1202021535D12, 1202021539P1

### 4. Test of effect of the Chinese medicine extraction liquid on cardiac muscle cells

### 1. Project briefs

This experiment adopts real-time cardiac muscle cell analysis system for test and assessment of cardiac safety of the Chinese medicine extraction liquid provided by Guizhou Chinese Medicine Co., Ltd. on cardiac muscle cells of first generation new born rat. The assessment parameters adopted for this experiment are in vitro cardiac muscle cell beating rate, beating amplitude, and beating rhythm and waveform subject to time dependence.

### 2. Background

### 2.1 Background of the study

ACEA Bioscience Co., Ltd. is a high technology biological company committed to R&D and manufacture of real-time cell detection instrument based on the impedance technology for which this company possesses autonomous patent. The ACEA micro-electrode sensor installed at bottom of micro-pore plate with innovative design can be used for real-time non-marking detection of cell status, and features high throughput, high accuracy, high sensitivity, and quantitative detection.

Cardiac functional damage due to heart disease or drug cardiac toxicity menaces human health and safety. It is a major challenge to heart researchers to deeply reveal mechanism of occurrence of major heart diseases, find new effective treatment measures based on this, and reveal cardiac toxicity of some drugs during new drug development. The real-time cardiac muscle cell analysis system (xCELLigence RTCA Cardio System) jointly developed by ACEA and Roche can dynamically monitor excitation contraction coupled action of cardiac muscle cells, detect and record cardiac muscle cell beating electrical signals for short/long periods in real-time. When a compound acts on cardiac muscle cells, affecting cell form and activity, or causing change of extent of opening of relevant channel, or change in contraction excitation conductivity, this real-time cardiac muscle cell analysis system can be used for real-time monitoring.

This system can be used to screen drugs that affect change of cardiac muscle cell contraction excitation conductivity, including ion channel and non-ion channel target point drugs. ACEA has used cardiac muscle cells induced by mouse embryo stem cells (CorAt, Axiogenesis) and those induced by human iPS cells to verify more than 50 types of known compounds. Slightly later, first generation rat cardiac muscle cells have been used for supplementary verification. Research results show that this real-time cardiac muscle cell analysis system can reliably and quantitatively test effect of cardiac arrhythmia compounds and non-ERG channel and voltage gating calcium channel compounds on cardiac muscle cell. Therefore, this system can be used as a tool to screen cardiac toxicity of compounds in early stage of drug development, for relevant drug high throughput screening and study of drug toxicity.

### Work contents:

The objective of this project is to use impedance detection system for detection and assessment of drug cardiac safety, and for assessment and appraisal of capability of compounds on change of first generation rat cardiac muscle cell beating rate.

In this project, each compound has been tested at 7 different concentrations. Before and after the test, different time points are selected to monitor cell beating status. Effect of the Chinese medicine extraction liquid on beating of first generation cardiac muscle cells is assessed through observation of the dose dependence of such liquid on first generation rat cardiac muscle cells.

### 2.2 Introduction to the system

This real-time cardiac muscle cell analysis system (xCELLigence RTCA Cardio System) inherits functions of the existing micro-electronics impedance detection systems (including RTCA SP, MP, and DP systems) based on cell of ACEA with autonomous intellectual property right, and can be used to functionally detect beating of cardiac muscle cells and cardiac cell toxicity of drugs. The core of this system is integration of micro-electronic cell sensors in arrays at bottom of a 96-well culture plate (Cardio-Plate 96). This greatly improves data acquisition rate. In addition to functions of long period detection of cell growth and proliferation similar to those of RTCA SP, MP, and DPRTCA systems, this cardiac muscle cell analysis system also features detection of beating of cardiac muscle cells formed by excitation contraction coupling.

RTCA cardiac muscle cell analysis system comprises the following (refer to the figure below):
- A: real-time cell analysis system control unit (RTCA Cardio Control Unit)
- B: real-time cell analyzer (RTCA Cardio Analyzer)
- C: cell detection station (RTCA Cardio Station)
- D: detection plate (Cardio-Plate 96)

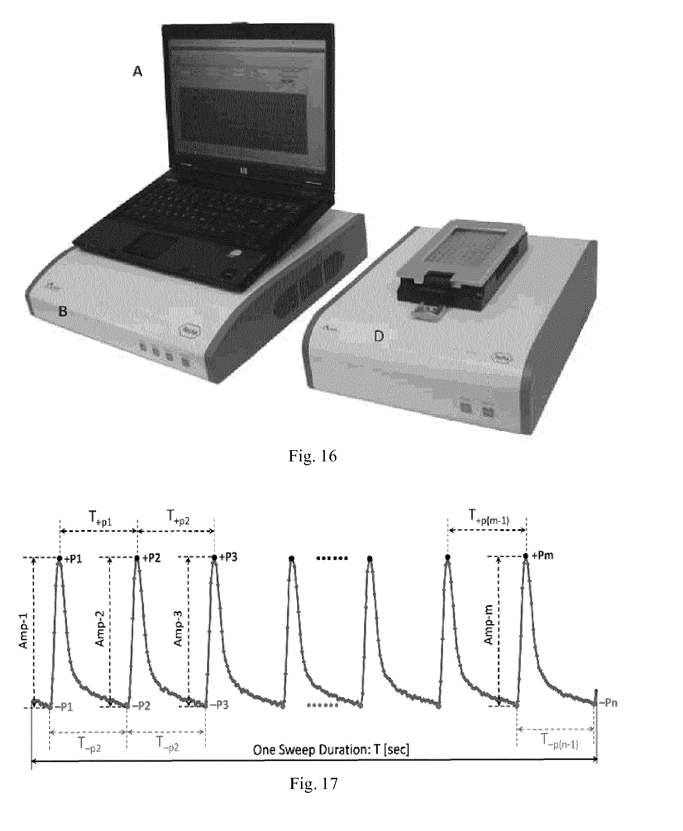

### (Refer to Fig.16)

Inoculate cells to the 96-well cardiac muscle cell detection plate to that these cells are attached to surfaces of electronic sensors. Place the RTCA Cardio Station in the CO₂ cell culture box (5% CO₂, 37°C) and the Cardio-Plate 96 on the RTCA Cardio Station, connected to the analyzer and the control unit. Through the detection station and the analyzer, impedance signals on sensor surfaces can be detected in real-time, and measured impedance signals are analyzed by the RTCA real-time cell analysis system control unit and Cardio software, to provide real-time quantitative biological status of cells, including quantity, activity, form, and kinetic change of cytoskeleton.

### First generation rat cardiac muscle cell

The first generation rat cardiac muscle cell model can be used for study of cardiac muscle cell form, biological study, electric physiological study, and pharmacological study. This model is a mature mode used for study of drug transport, toxicity, and electric physiological characteristics. Take 10 new born (within 24h) rats, sterilize using 75% ethanol, and then cut center of sternum with scissors. Pay attention to avoid cutting the alimentary tract, to prevent contamination. Carry out aseptic cutting and taking of the ventricle part, carefully strip cardiac atrium and large blood vessel tissues, quickly put it in pre-cooled serum-free DMEM, wash it for 3 times to remove residual blood cells, cut it to tissue blocks of the size of 0.5 mmx 0.5 mmx 0.5mm, place them in a small glass bottle, and add 1.5 ml of 0.07% parenzyme and 0.07% collagenase I for digestion. Collect supernatant after digestion, add suitable amount (equal to the volume of the supernatant) of DMEM culture medium containing 10% of fetal calf serum to terminate the action of the digestive enzyme. Repeat above digestion steps till tissue blocks disappear. Use 200 mesh sieve to remove undigested tissues by filtering. Centrifuge the supernatant for 5min (at 800 r/min). Discard the supernatant and use DMEM culture medium containing 10% of fetal calf serum to blow off precipitated cells. Inoculate the cells in a culture flask and place this flask in CO₂ cell incubator (5% CO₂, 37°C) for standstill culture for 60 min (sticking to wall at differential speed). Collect cells that do not stick to wall and inoculate them to the 96-well cardiac muscle cell detection plate treated by gelatin at the density of 15000 cells per well. Place the plate on the Cardio system for detection.

### 3. Experiment design and scheme

### 3.1 Experiment instruments:

This experiment adopts real-time cardiac muscle cell analysis system jointly developed by ACEA and Roche.

| Product Name | Cat. No. | Description |
|---|---|---|
| RTCA Cardio Station | 6417019001 | RTCA Cardio Station for screening of cardiomyocytes |
| RTCA Cardio Analyzer | 6416993001 | RTCA HT Analyzer (incl. Power Cable and Serial Cable) |
| RTCA Cardio Control Unit | 6200184001 | Basic Notebook HP 6930p, pre-installed RTCA Cardio Software |
| E-Plate Cardio 96 | 6417051001 | 1X6 plates |
| E-Plate Cardio 96 | 6417035001 | 6X6 plates |

### 3.2 Experiment reagents and consumables:

DMEM culture solution: goods No.: SH30022.08B, Hyclone (China)
Serum: goods No.: 16000-044, Gibco (USA)
Pancreatic enzyme: goods No.: 27250-018, Gibco (USA)
Collagenase: goods No.: 17101-015, Gibco (USA)
DMSO: goods No.: D2650, Sigma (USA)
HBSS solution: 14175, Gibco (USA)
Gelatin: goods No.: 48732, Sigma (USA)
PBS: goods No.: SH30256.01B, Hyclone (China)

### 3.3 Method of drug preparation

According to information provided by client, 125 ml of medical juice of Chinese medicine extraction liquid is obtained from 20 g of the drug, i.e. a concentration of 160 mg/ml. During the experiment, this is diluted by culture medium according to actual demand, and then added into the cell.

### 3.4 Experiment design and scheme

Culture of first generation new born rat cardiac muscle cells: Inoculate 17000 cells per well. After inoculation, place them in the real-time cardiac muscle cell analysis system for detection using steps and parameters given below (refer to Table of setup of acquired signals). After 48h, replace the liquid. 2h after liquid replacement when cell signals are stable, add the cells into the cell wells according to corresponding compound concentration. After dosing, return the cells to the detection system for real-time acquisition of cardiac muscle cell beating signals. Each compound shall adopt two or more composite wells.

| | | C2 | C3 | C4 | C5 | C6 | C7 | C8 | C9 | C10 | C11 |
|---|---|---|---|---|---|---|---|---|---|---|---|
| A | | | | | | | | | | | |
| B | Chinese Medicine Extraction Liquid | 7.32 µg/ml | 21.95 µg /ml | 65.84 µg/ml | 0.20 mg/ml | 0.59 mg/ml | 1.78 mg/ml | 5.33 mg/ml | 5.33 mg/ml | 16.00 mg/ml | CON |
| C | | | | | | | | | | | |

### Acquisition signals setup is as follows:

| Step | Number of cycles | Interval/time | Monitoring time/time | Remark |
|---|---|---|---|---|
| 0 | 10 | 1 min | 20s | Measure base line |
| 1 | 48 | 1 hour | 20s | Cell sticking to wall/growth |
| 2 | 8 | 15 min | 20s | Liquid replacement |
| 3 | 10 | 1 min | 20s | Before dosing |
| 4 | 60 | 1 min | 20s | After dosing |
| 5 | 12 | 5 min | 20s | |
| 6 | 100 | 15 min | 20s | |
| 7 | 50 | 1 hour | 20s | |

### 3.5 Data analysis

3.5.1 Calculate cell bearing rate at each time point and plot curve of beating rate vs time)

### 3.5.2 Parameters for analysis include:

- Time dependent beating rate
- Normalized beating rate
- Time dependent amplitude
- Normalized amplitude
- Overall cell activity before and after dosing (Cell Index)
- Time dependent beating rhythm irregularity
- Time dependent beating similarity

### Definitions of analysis parameters

*Each beat of cell corresponds to one cardiac muscle cell excitation contraction coupling. Such beats include Positive Peaks (+P) and Negative Peaks (-P) that occur in sequence. Number of positive peaks and number of negative peaks that occur in unit time reflect cardiac muscle cell beating characteristics and cell status (refer to* *Fig. 17**).*

*Description of cardiac muscle cell beating modes and main relevant parameters:*

### Amplitude:

*Difference of cell index between negative peak and positive peak (Amp-1, Amp-2, Amp-3,* ..., *Amp-m shown in the figure)*

### Beating Period:

*As shown in above figure, in one sweep duration, there are m positive peaks (*+*P1, +P2,* +*P3,...,* +*Pm) and n negative peaks (-P1, -P2, -P3,* ..., *-Pn). The time between one positive peak and one negative peak is defined as the beating period*.

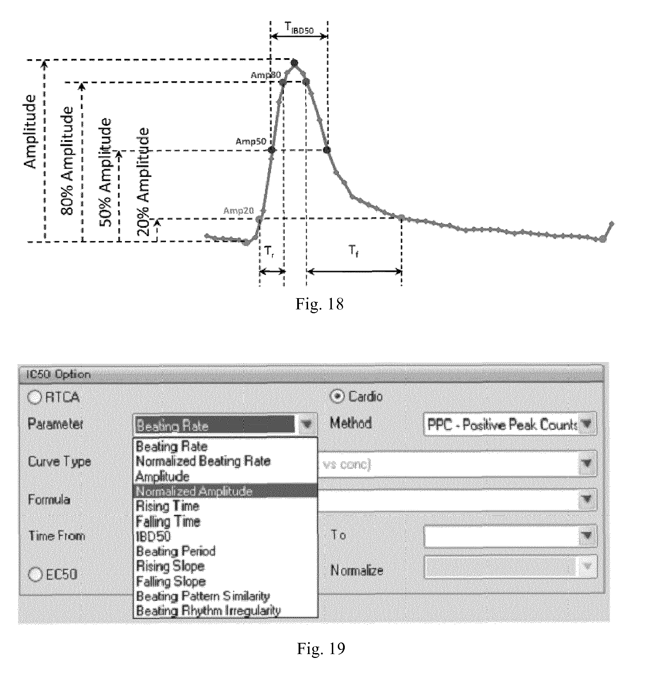

*For each beat, parameters Rising Time, Falling Time, IBD50, Rising Slope, and Falling Slope etc. are used to describe beating rising process and falling process (**Fig. 18**).*

### 3.5.3 Method of analysis of parameters and data in cardiac muscle cell model

RTCA cardiac muscle cell analysis software provides 12 parameters used for analysis of cardiac muscle cell beating characteristics. In this report, suitable parameters are selected for analysis (Fig. 19).

| Parameter | Unit | Method | Description of parameters |
|---|---|---|---|
| Beating Rate | beatings/ min | Positive Peak Counts | Number of all positive peaks in 1min |
| | | Negative Peak Counts | Number of all negative peaks in 1min |
| | | Positive Peak Period Based | Calculate each beating rate between positive-positive peak |
| | | Negative Peak Period Based | Calculate each beating rate between negative-negative peak |
| Normalized Beating Rate | N/A | Positive Peak Counts | When normalized beating rate is selected, a pull-down menu will appear. In this menu, select normalized time point. Normalized |
| | | Negative Peak Counts | |
| | | Positive Peak Period Based | beating rate is the ratio of beating rate at selected time point to beating rate at the normalized time point. |
| | | Negative Peak Period Based | |
| Amplitude | Same as Cell Index | WP-Whole Peak | Amplitude from one negative peak to the next positive peak |
| | | PP-Positive Peak | Amplitude from base line to positive peak |
| | | NP-Negative Peak | Amplitude from base line to negative peak |
| Normalized Amplitude | N/A | WP-Whole Peak | When normalized amplitude is selected, a pull-down menu will appear. In this menu, select normalized time point. Normalized amplitude is the ratio of amplitude at selected time point to amplitude at the normalized time point. |
| | | PP-Positive Peak | |
| | | NP-Negative Peak | |

### 4 Experiment results

The Chinese medicine extraction liquid only affects cell activity at the concentration of 16mg/ml, not at other concentrations. Effect of this extraction liquid on beating of cardiac muscle cells is: there is no apparent effect on beating rate, amplitude, and rhythm of rat first generation ventricle muscle cells in a short period, but certain cardiac toxic action exists on ventricle muscle cells after a relatively long period of action. In particular, after of 2 hours of action under high concentration (16 mg/ml), cardiac muscle cell beating rate may be lowered, and beating may totally stop after 6 hours of action.

2-12h after addition of low concentration (5.33 and 1.78 mg/ml) drug, cardiac muscle cell beating rate is slightly lowered and the amplitude increases. At 12h, beating is restored to normal.

At concentration lower than 0.59 mg/ml, the drug has no apparent effect on beating of cardiac muscle cells.

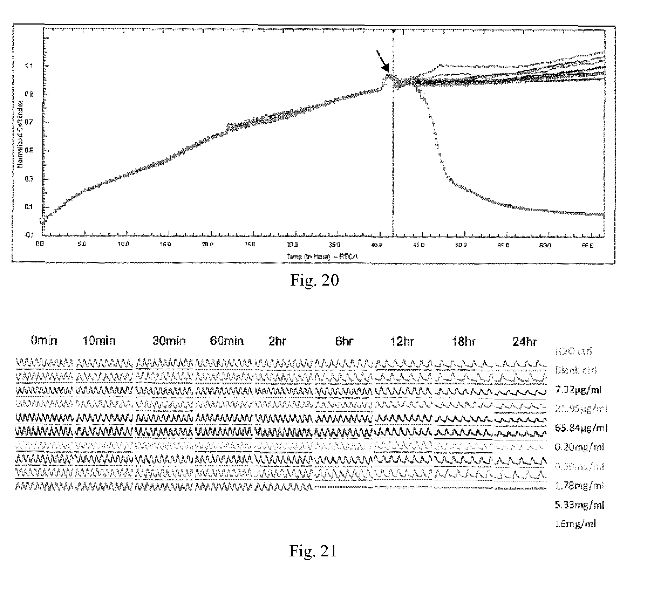

### 4.1 Effect of the Chinese medicine extraction liquid on activity of new born rat first generation cardiac muscle cells (Fig. 20)

Chinese medicine extraction liquid: (H20 ctrl, blank ct, 7.32 ug/ml, 21.95 ug/ml, 65.84 ug/ml, 0.20 mg/ml, 0.59 mg/ml, 1.78 mg/ml, 5.33 mg/ml, 16.00 mg/ml)

Dynamic detection of suppression of activity of first generation cardiac muscle cells by the Chinese medicine extraction liquid: The above figure is real-time dynamic diagram of cell growth and drug effect. In this figure, abscissa indicates time of cell culture and drug action, and the ordinate indicates NCI. The NCI prompts cell growth status: the higher is this value, the more living cells. The arrow points to dosing time. Dosing concentration is shown in this figure. This figure shows drug concentration dependence dynamic response curve of cells under drug action. ctrl is negative comparison and shows normal cell growth dynamic curve. (Note: In H20 ctrl comparison well, add 10% H20 (same as amount of original liquid of highest concentration drug)).

It can be seen from results of test of effect of the Chinese medicine extraction liquid on new born rat first generation cardiac muscle cell that when high concentration (16 mg/ml) drug is used, after a period of time, the cell index falls quickly, reflecting apparent cytotoxicity of high concentration drug, which may cause cell morphologic change, poor sticking on wall, and even death. When drug concentration is lower than 5.32 mg/ml, there is no apparent effect of the drug on cell activity. However, drug of this concentration may cause certain change in beating rate and amplitude of cardiac muscle cells.

### 4.2 Change of beating curve of first generation cardiac muscle cell with time after dosing (Fig. 21)

### 4.3 Summary of toxic action of the Chinese medicine extraction liquid on new born rat first generation cardiac muscle cell

| (µg/ml) | 1600 | 5330 | 1780 | 590 | 200 | 65.84 | 21.95 | 7.32 | CON |
|---|---|---|---|---|---|---|---|---|---|
| Before treatment | √ | √ | √ | √ | √ | √ | √ | √ | √ |
| 10 min | √ | √ | √ | √ | √ | √ | √ | √ | √ |
| 30 min | √ | √ | √ | √ | √ | √ | √ | √ | √ |
| 1h | √ | √ | √ | √ | √ | √ | √ | √ | √ |
| 2h | √ | √ | √ | √ | √ | √ | √ | √ | √ |
| 6h | XX | X | X | √ | √ | √ | √ | √ | √ |
| 12h | XX | X | X | √ | √ | √ | √ | √ | √ |
| 18h | XX | X | X | √ | √ | √ | √ | √ | √ |
| 24h | XX | X | X | √ | √ | √ | √ | √ | √ |

| | | | | | | | | | |
|---|---|---|---|---|---|---|---|---|---|
| Note: Effect of the drug on beating of cell: XX indicates no beating; X indicates apparent irregularity; and V indicates normal beating. | | | | | | | | | |

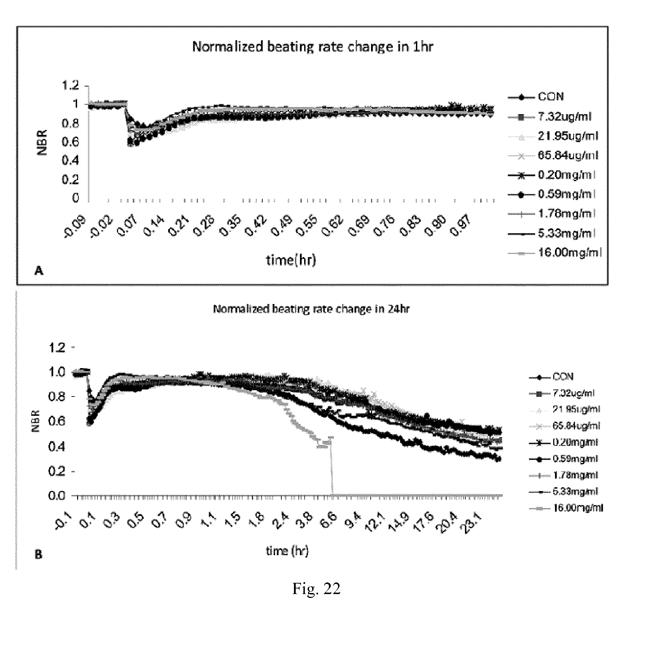

### 4.4 Effect of the Chinese medicine extraction liquid on beating rate (Fig. 22)

This figure shows quantity/effect relation of change of cardiac muscle cell beating rate dependent on Chinese medicine extract drug concentration and time. The parameter used is Normalized Beating Rate (NBR). Parameter calculation method: ratio of the beating rate at selected time point to the beating rate at standardized time point (0 min).

In this figure, part A shows the process of change of cell beating rate within 1h after dosing. Party B shows full process of change of cell beating rate from 10 min before dosing to 24h after dosing.

It can be seen from this figure that within 10min before dosing, cell beating rate is consistent and has good homogeneity. After dosing, there is no apparent effect on cell beating by the drug in a short period. After 2 hr, the high concentration drug causes lowering of cell beating rate, till full stop of beating at 6hr after dosing. Drug of 5.33 mg/ml causes slightly lowered beating rate in a period of time. Drug of concentration less than 1.78 mg/ml has no apparent effect on cell beating. This indicates that high concentration of this drug has apparent effect on heart beating, but low concentration of this drug has no serious effect on heart beating.

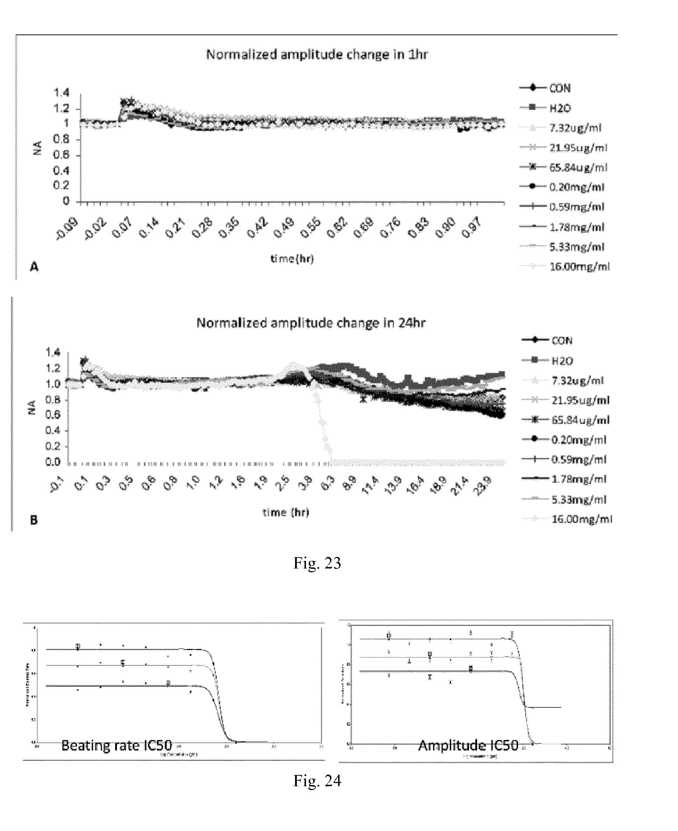

### 4.5 Effect of the Chinese medicine extraction liquid on beating amplitude (Fig. 23)

This figure shows change of cardiac muscle cell beating amplitude dependent on drug concentration and time. The parameter used is Normalized Amplitude (NA). Parameter calculation method: ratio of the amplitude at selected time point to the amplitude at standardized time point (0 min).

In this figure, part A shows process of change of cell beating amplitude within 1h after dosing, and part B shows full process of change of cell beating amplitude from 10min before dosing to 24h after dosing.

It can be seen from this figure that within 10 min before dosing, cell beating amplitude is stable. In a short period after dosing, the drug has no apparent effect on the amplitude. 2 hr after dosing, high concentration (16 mg/ml) drug causes lowered cell beating rate, till full stop of beating at 6hr after dosing. 5.33 mg/ml drug causes slightly lowered beating rate. When drug concentration is lower than 1.78 mg/ml, there is no apparent effect on cell beating. This indicates that high concentration of this drug has apparent effect on heart beating, while low concentration of this drug has no serious effect on heart beating.

### 4.6 IC50 of effect of the Chinese medicine extraction liquid on new born rat first generation cardiac muscle cell beating rate and amplitude (Fig. 24)

IC50 fitting curves (beating rate/amplitude) of 4 time points in above figure

| Time after dosing | Beating rate | | Amplitude | |
|---|---|---|---|---|
| | IC50 (mg/ml) | Square R | IC50 (mg/ml) | Square R |
| 6hr | 6. 88 | 0.985 | 9. 88 | 0.983 |
| 12hr | 7.00 | 0.990 | 10.65 | 0.987 |
| 24hr | 6.68 | 0.970 | 7. 72 | 0.539 |

IC50 values of cell beating rate and amplitude calculated at 3 time points

### 5. Conclusions of the experiment, possible questions, and recommendations

5.1 High concentration (16 mg/ml) of this drug quickly affects cell activity, causing death of cells.
5.2 This drug affects beating rate and amplitude of first generation new born rat cardiac muscle cell with concentration dependence. High concentration (16 mg/ml) of this drug may cause stop of beating at about 6 hr after dosing, while medium concentration (5.33 mg/ml; 1.78 mg/ml) of this drug may cause slightly lowered beating rate and slightly increased amplitude of the cells at about 6 hr after dosing. This indicates that high concentration of this drug apparently affects heart beating while low concentration of this drug has no serious effect on heart beating.
5.3 Cardiac muscle cell beating waveform related to specificity ion channel caused by this drug is not seen.

The above results are for reference only. Particular mechanism needs further screening and research.

### Experiment records

Save in ACEA Biosciences (Hangzhou) Co., Ltd. client database
No.: 1112121715CD.

| PCT | Print (original electronic form) | |
|---|---|---|
| 0-1 | Form PCT/RO/134 the indications relate to the deposited microorganism or other biological | |
| 0-1-1 | material (PCT Rule 13.2) | |
| | so ft vision | PCT-FAFE 01/0.20.5.19 |
| International application No. | PCT/CN2012/086605 | |
| Applicant's or agent's file reference | ZB12-04F | |
| | | |
| 1 | The indications made below relate to the deposited microorganism or other biological material referred to in the description | |
| 1-1 | page | 3 |
| 1-2 | Line Number | 25 |
| 1-3 | Depositary matters | |
| 1-3-1 | Name of depositary institution | Collection Name CCTCC China Center for Type Culture Collection |
| 1-3-2 | Address of depositary institution | China, Wuhan University, Wuhan City, Hubei Province, Postal Code: 430072, Hubei (CN). |
| 1-3-3 | Date of deposit | April 2, 2011 (02 Apr. 2011) |
| 1-3-4 | Accession No. | CCTCC P201102 |
| 1-5 | This note is for following designated countries | all designated States |

| For receiving Office use only | | |
|---|---|---|
| 0-4 | This sheet was received with the international application: (Yes or No) | Yes |
| 0-4-1 | authorized officer | LI li |

| For International Bureau use only | | |
|---|---|---|
| 0-5 | This sheet was received by the International Bureau on | |
| 0-5-1 | authorized officer | |

## Claims

1. A type of medical combination containing natural human epoxidase and natural doxorubicin or para-doxorubicin, wherein the raw material of this medical combination is treasures grass.

2. The medical combination containing natural human epoxidase and natural doxorubicin or para-doxorubicin according to claim 1, wherein "The raw material of this medical combination is treasures grass" means that powder obtained from dewatering, drying, and crushing of whole treasures grasses or extract of treasures grasses is adopted.

3. The medical combination containing natural human epoxidase and natural doxorubicin or para-doxorubicin according to claim 2, wherein said extract of treasures grass refers to one of the following:
Powder obtained from concentration and drying of treasures grass water decoction liquid;
Alcohol extraction liquid of treasures grass water decoction liquid obtained by alcohol extract, or powder obtained from concentration and drying of the same;
Extract obtained from organic extraction of whole treasures grass or treasures grass water decoction liquid or powder obtained from re-concentration and drying of the same;
Extract obtained from CO₂ supercritical extraction of whole treasures grass or treasures grass water decoction liquid, or powder obtained from re-concentration and drying of the same;
Volatile oil extracted from whole treasures grass or treasures grass water decoction liquid.

4. The medical combination containing natural human epoxidase and natural doxorubicin or para-doxorubicin according to claim 1, 2, or 3, wherein said medical combination containing natural doxorubicin and human epoxidase also includes auxiliary materials permitted in the field of pharmacy.

5. The medical combination containing natural human epoxidase and natural doxorubicin or para-doxorubicin according to claim 4, wherein auxiliary materials permitted in the field of pharmacy refer to: excipient, condiment, or antioxidant used to make tablets; antioxidant or stabilizer used to make capsules; condiment, stabilizer, or antioxidant used to make oral liquid or syrup; emulsifier or vaseline used to make externally applied medicinal extract; and emulsifier, solvent, or stabilizer used to make injection.

6. Method of preparation of the medical combination containing natural human epoxidase and natural doxorubicin or para-doxorubicin according to claim 1, wherein the steps are:
(1) Cleaning of whole treasures grass;
(2) Dewatering, drying, and crushing of the clean whole treasures grass obtained in Step (1) to yield a powder; said powder has grain size of 125um or 5.8um, or passes 120 mesh sieve;
Said step (2) can also adopt treasures grass extract obtained by one of the following:
(2) -1 Treasures grass water decoction liquid or powder obtained by re-concentration and drying of this liquid;
(2) -2 Alcohol extraction liquid of treasures grass water decoction liquid obtained by alcohol extract, or powder obtained by re-concentration and drying of the same;
(2) -3 Extract of whole treasures grass or treasures grass water decoction liquid obtained by organic extraction, or powder obtained by re-concentration and drying of this extract;
(2) -4 Extract obtained by CO₂ supercritical extraction of whole treasures grass or treasures grass water decoction liquid, or powder obtained by re-concentration and drying of this extract;
(2) -5 Volatile oil extracted from whole treasures grass
(3) Mix powder and/or volatile oil obtained in step (2), (2) -1, (2) -2, (2) -3, (2) -4, or (2) -5 with medical auxiliary substance to prepare required dose pattern;
(4) Sterilization;
(5) Analysis;
(6) Packing.

7. Application of the medical combination containing natural human epoxidase and natural doxorubicin or para-doxorubicin according to claim 1 in preparation of drugs to treat nephritis.

8. Application of the medical combination containing natural human epoxidase and natural doxorubicin or para-doxorubicin according to claim 1 in preparation of drugs to treat cholecystitis and gall bladder polypus.

9. Application of the medical combination containing natural human epoxidase and natural doxorubicin or para-doxorubicin according to claim 1 in preparation of drugs to treat tumor.

10. Application of the medical combination containing natural human epoxidase and natural doxorubicin or para-doxorubicin according to claim 1 in preparation of anti-inflammation and painkiller drugs.
